(19) Europäisches Patentamt
European Patent Office
Office européen des brevets

(11) **EP 4 137 809 A1**

(12) **EUROPEAN PATENT APPLICATION**

(43) Date of publication:
**22.02.2023 Bulletin 2023/08**

(21) Application number: **22190876.7**

(22) Date of filing: **17.08.2022**

(51) International Patent Classification (IPC):
*G01N 27/333* (2006.01)    *G01N 33/18* (2006.01)
*G01N 27/416* (2006.01)    *G01N 27/42* (2006.01)

(52) Cooperative Patent Classification (CPC):
G01N 27/333; **G01N 27/4166; G01N 27/423;**
**G01N 33/182**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **17.08.2021   US 202163260329 P**

(71) Applicant: **McMaster University
Hamilton, Ontario L8S 4L8 (CA)**

(72) Inventors:
• **SELVAGANAPATHY, Ponnambalam Ravi
  Dundas, Ontario (CA)**
• **PATEL, Vinay
  Hamilton, Ontario (CA)**

(74) Representative: **Hamer, Christopher K. et al
Mathys & Squire
The Shard
32 London Bridge Street
London SE1 9SG (GB)**

(54) **APPARATUS AND METHOD FOR MEASURING PHOSPHATE IN WATER**

(57)    Apparatus and methods are provided for measuring phosphate in water. The methods can involve electrically pre-treating a potentiometric sensor and using the electrically pretreated potentiometric sensor in a water sample to obtain a phosphate measurement. The potentiometric sensor includes a working electrode and a counter electrode. The working electrode includes a metal/metal oxide electrode having a detection surface. The electrical pre-treatment generates a metal phosphate on the detection surface of the metal/metal oxide electrode. The phosphate measurement results in a fresh layer of mixed oxide on the detection surface of the metal/metal oxide electrode. The electrically pre-treatment can be performed in situ in the water sample, allowing for field monitoring of water bodies. In some embodiments, the method can also involve cleaning the sensor prior to electrically pre-treating the sensor or after obtaining the phosphate measurement.

200

Electrically pre-treat a potentiometric sensor — 210

Use the electrically pre-treated potentiometric sensor in a water sample to obtain a phosphate measurement — 220

Clean the sensor — 230

**FIG. 2**

**EP 4 137 809 A1**

**Description**

**Cross Reference to Related Applications**

This application claims the benefit of U.S. Provisional Patent Application No. 63/260,329, filed on August 17, 2021. The entire contents of U.S. Provisional Patent Application No. 63/260,329 are hereby incorporated by reference for all purposes.

**Field**

The described embodiments relate to potentiometric sensors. In some example embodiments, the devices and methods can relate to measuring phosphate.

**Background**

Phosphate is an important analyte to monitor in various water bodies. Phosphate is a major pollutant responsible for algal bloom in various water bodies, including lakes and ponds. Elevated levels of phosphorus results in eutrophication, which is lethal to aquatic life and disturbs the ecological balance in water bodies.

To avoid pollution and to maintain optimal water quality, phosphate is treated in wastewater before disposal into natural water sources. Agricultural run offs are a major source of phosphorus in the wastewater. Current methods for phosphate monitoring are laboratory based. However, the transportation of samples from the field to laboratories has significant costs, which restricts widespread monitoring. On-site periodic field monitoring has relatively lower costs, which allows for more widespread monitoring, resulting in more efficient use of fertilizers and reduction of phosphate run off. Field monitoring can also reduce the variability associated with sample collection, transportation, and sample degradation effects compared to laboratory-based monitoring.

**Summary**

The various embodiments described herein generally relate to methods (and associated apparatus) for measuring phosphate in water. The methods involve electrically pre-treating a potentiometric sensor and using the electrically pre-treated potentiometric sensor in a water sample to obtain a phosphate measurement. The potentiometric sensor includes a working electrode and a counter electrode. The working electrode includes a metal/metal oxide electrode having a detection surface. The pre-treatment generates a metal phosphate on the detection surface of the metal/metal oxide electrode. The phosphate measurement results in a fresh layer of mixed oxide on the detection surface of the metal/metal oxide electrode.

In at least one embodiment, the metal/metal oxide electrode can include at least one selected from a group consisting of a cobalt/cobalt-oxide electrode, a molybdenum/molybdenum oxide electrode, and a tungsten/tungsten oxide electrode.

In at least one embodiment, the mixed oxide can include at least one selected from a group consisting of metal hydroxides, metal oxy hydroxides, and metal oxides.

In at least one embodiment, the method can involve electrically pre-treating the potentiometric sensor in situ in the water sample.

In at least one embodiment, the method can involve electrically pre-treating the potentiometric sensor in an analyte solution.

In at least one embodiment, the method can involve applying an anodic current between the working electrode and the counter electrode.

In at least one embodiment, the anodic current can be a pulse having pre-determined pulse duration.

In at least one embodiment, the method can involve the pre-determined pulse duration can be between 150 seconds to 250 seconds.

In at least one embodiment, the pre-determined pulse duration can be about 220 seconds.

In at least one embodiment, the anodic current can have a density between about -1 mA/cm$^2$ to about - 20 mA/cm$^2$.

In at least one embodiment, the anodic current can have a density of about -9.18 mA/cm$^2$.

In at least one embodiment, the method can involve using the potentiometric sensor in an open circuit mode for at least a pre-determined measurement duration to obtain the measurement.

In at least one embodiment, the pre-determined measurement duration can be about 110 seconds.

In at least one embodiment, the method can further involve removing mixed oxide from the detection surface prior to electrically pre-treating the sensor.

In at least one embodiment, the method can further involve removing mixed oxide from the detection surface after obtaining the phosphate measurement.

In at least one embodiment, the method can involve chemically dissolving mixed oxide from the detection surface.

In at least one embodiment, the method can involve using sandpaper to mechanically remove mixed oxide from the detection surface.

In at least one embodiment, the sandpaper can include silicon carbide.

In at least one embodiment, the method can involve removing at least a portion of a passivation layer on the metal/metal oxide electrode to increase the detection surface.

In at least one embodiment, the counter electrode can include stainless-steel.

In at least one embodiment, the potentiometric sensor can further include a reference electrode.

In at least one embodiment, the reference electrode can include double junction silver-silver chloride.

In at least one embodiment, the method can further involve obtaining a dissolved oxygen concentration of the water.

In another broad aspect, an apparatus for field monitoring of water is disclosed. The apparatus can include a potentiometric sensor operable to measure phosphate in situ in a water sample. The potentiometric sensor can include a working electrode and a counter electrode. The working electrode can include a metal/metal oxide electrode. The apparatus can also include an electrical source connectable to the working electrode and the counter electrode for applying an anodic current prior to measuring the phosphate in situ in the water sample.

In at least one embodiment, the metal/metal oxide electrode can include at least one selected from a group consisting of a cobalt/cobalt-oxide electrode, a molybdenum/molybdenum oxide electrode, and a tungsten/tungsten oxide electrode.

In at least one embodiment, the counter electrode can include stainless-steel.

In at least one embodiment, the potentiometric sensor can further include a reference electrode.

In at least one embodiment, the reference electrode can include double junction silver-silver chloride.

In another broad aspect, a kit for field monitoring of water is disclosed. The kit can include an apparatus for field monitoring of water and sandpaper for polishing a potentiometric sensor of the apparatus.

In at least one embodiment, the sandpaper can include silicon carbide.

**Brief Description of the Drawings**

Several embodiments will now be described in detail with reference to the drawings, in which:

FIG. 1A is an illustration of an overview of the process for fabricating a potentiometric phosphate sensor, in accordance with at least one embodiment;

FIG. 1B is an illustration of an example setup for obtaining a measurement using a potentiometric sensor, in accordance with at least one embodiment;

FIG. 2 is a flowchart of a method for measuring phosphate in water, in accordance with at least one embodiment;

FIG. 3A is an illustration of the chemical reaction of the sensing mechanism of a cobalt-based sensor without electrical pre-treatment;

FIG. 3B is an illustration of the chemical reaction of the sensing mechanism of a cobalt-based sensor with electrical pre-treatment, in accordance with at least one embodiment;

FIG. 3C is a graph comparing the stability of cobalt-based sensors without electrical pre-treatment and with electrical pre-treatment, in accordance with at least one embodiment;

FIG. 3D is Nyquist plot comparing the electrochemical impedance of cobalt-based sensors prior to sensing, after sensing without electrical pre-treatment, and after sensing with electrical pre-treatment in accordance with at least one embodiment;

FIG. 3E is schematic representation of a Randles circuit, to which the electrochemical impedance results of FIG. 3D were fitted to;

FIG. 3F is a graph comparing the potential response of cobalt-based sensors with electrical pre-treatment, in accordance with at least one embodiment, and without electrical pre-treatment;

FIG. 4A is a graph comparing the potential response of cobalt-based sensors with electrical pre-treatment applied for different time durations, in accordance with at least one embodiment;

FIG. 4B is a graph comparing the sensitivity of cobalt-based sensors with electrical pre-treatment applied for different time durations, in accordance with at least one embodiment;

FIG. 5A is a graph comparing the dynamic response of cobalt-based sensors to different phosphate solutions, in accordance with at least one embodiment;

FIG. 5B is magnified view of a portion of the graph of FIG. 5A;

FIG. 6 is a Nyquist plot comparing the electrochemical impedance of cobalt-based sensors in different phosphate solutions, in accordance with at least one embodiment;

FIG. 7 is a graph illustrating repeated measurements using a cobalt-based sensor, in accordance with at least one embodiment;

FIG. 8A is a graph comparing the potential response of cobalt-based sensors to solutions having different conductivities, in accordance with at least one embodiment;

FIG. 8B is a graph comparing the potential response of cobalt-based sensors to solutions having different dissolved oxygen concentrations, in accordance with at least one embodiment;

FIG. 8C is a graph comparing the potential response of cobalt-based sensors to solutions having different pH levels, in accordance with at least one embodiment;

FIG. 8D is a graph comparing the potential response of cobalt-based sensors to solutions having different interferents, in accordance with at least one embodiment; and

FIG. 9 is a graph comparing the potential response of cobalt-based sensor to different water samples, in accordance with at least one embodiment.

The drawings, described below, are provided for purposes of illustration, and not of limitation, of the aspects and features of various examples of embodiments described herein. For simplicity and clarity of illustration, elements shown in the drawings have not necessarily been drawn to scale. The dimensions of some of the elements may be exaggerated relative to other elements for clarity. It will be appreciated that for simplicity and clarity of illustration, where considered appropriate, reference numerals may be repeated among the drawings to indicate corresponding or analogous elements or steps.

**Description of Example Embodiments**

Phosphate detection can be performed using optical and electrochemical methods. Optical methods are more common and can be laboratory based or implemented in online monitoring systems. Optical methods involve detecting and quantifying a color change of a reagent (e.g., molybdenum blue or vanadate/molybdate yellow) upon reaction with phosphate in an acidic medium. Such colorimetric methods provide accurate measurements but require toxic reagents, sophisticated instrumentation, sample preparation, and skilled personnel. Although some commercial online phosphorus monitoring systems use such colorimetric methods, these systems are expensive, large in dimensions, and require on-site chemical and waste storage systems.

Electrochemical sensors are lower in cost, require less complex instrumentation, and are more amenable for field use. Traditionally, electrochemical sensing of phosphates is performed using potentiometric, amperometric, and voltametric sensors. Amperometric sensors require multiple enzymes or high alkaline pH (-11), which is difficult for in-field monitoring. Voltametric "tongue" type sensors for phosphate measurements require an electrode panel of multiple electrodes, a reliable training dataset to predict reliable phosphate concentrations, and are limited to a narrow range of 0.54 - 7.3 ppm. Furthermore, the requirement for a training dataset limits its application to specific locations of sample types. Another method, cyclic voltammetry, detects phosphate using phosphomolybdate ions and can measure a relatively larger range of 0.25 to 3.08 mg/l, which works well with soil. However, the cyclic voltammetry requires a low pH (pH =1) to avoid silicate interference and is limited to a narrow range for other environmental samples.

Potentiometric sensors have the advantage of being solid-state, low cost and reagent free. Potentiometric sensors use two different approaches: (1) using metal/metal oxide electrodes or (2) using ion selective electrodes. The ion selective approach has limitations as a suitable and selective ionophore capable of detecting phosphate in the presence of other common interferents is still under development. Potentiometric metal/metal oxide electrode sensors require extensive pre-conditioning. Also, most of the reported sensitivity for potentiometric sensors ranges from -19 mV/decade to -54 mV/decade for metal-based sensors and -28 mV/decade to -55.7 mV/decade for ion selective membrane-based sensors.

Cobalt/Cobalt oxide sensors, that is, cobalt-based sensors, are the most common metal/metal oxide sensors used for phosphate detection. Cobalt-based sensors have a quick response time and are easy to fabricate. However, cobalt-based sensors have lower sensitivity, limited dynamic range, and require a chemical pre-conditioning in a standard solution before measurement.

The standard solution used for chemical pre-conditioning of cobalt sensors can be deionized water (i.e., "DI water") and/or a buffer with a defined phosphate concentration. The pre-conditioning time can vary from five minutes for microfabricated electrodes to more than two hours for wire-based electrodes. For example, the pre-conditioning for a planar microfabricated electrode can be a two-step process in which the electrode is first immersed into DI water for 2 minutes followed by the electrode being immersed in $10^{-4}$ M dihydrogen phosphate solution for 200 seconds.

Many of the existing methods for using cobalt-based sensors involve measurements being taken in a phthalate buffer, which is not ideal for field sample measurements. As well, most of the existing cobalt-based solid state potentiometric sensors use two-electrode configurations (e.g., a working electrode and a reference electrode). However, the accuracy of two-electrode configurations can decline due to drift. A three-electrode configuration for a sensor can include a counter electrode to maintain a cathodic potential between the working electrode and the counter electrode to prevent surface passivation of the cobalt during long term water exposure.

The various embodiments described herein generally relate to methods (and associated apparatus to implement the method) for using potentiometric sensors without chemical pre-conditioning. In particular, the methods can involve the use of cobalt-based potentiometric sensors for measuring phosphate in water.

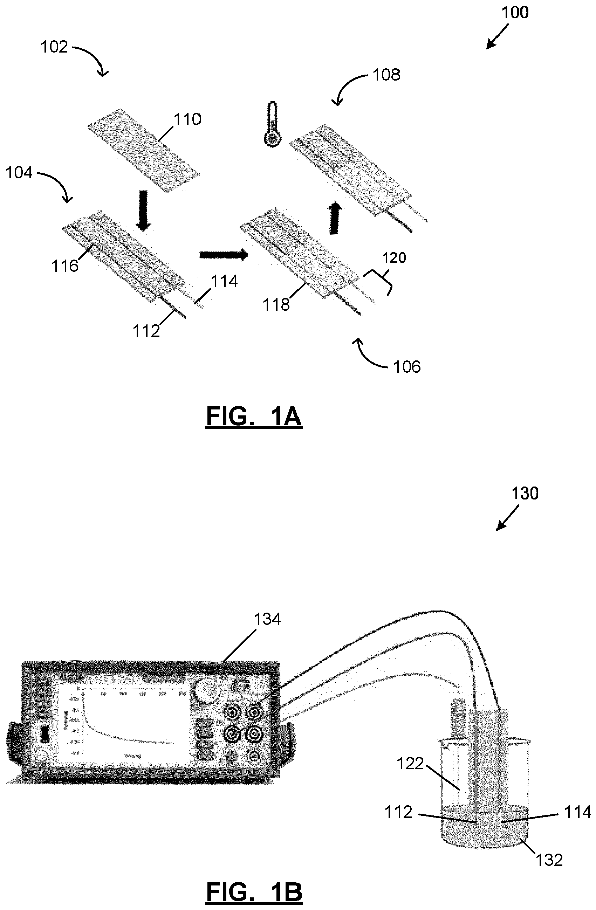

Reference now is made to FIG. 1A, which illustrates an overview of the process 100 for fabricating a potentiometric phosphate sensor, in accordance with at least one embodiment. The process 100 can begin at 102 with a glass substrate 110. The glass substrate 110 can be a standard, plain microscopic glass slide.

At 104, a working electrode 112 and a counter electrode 114 are attached to the glass substrate 110. The electrodes can be attached to the glass substrate 110 using a double-sided conductive tape 116, such as a double-sided copper tape. in some embodiments, a cobalt wire (Co) can be provided as the working electrode 112 and a stainless-steel (SS) wire can be provided as the counter electrode 114.

At 106, a passivation layer 118 can be applied to a portion of the sensor. In some embodiments, the passivation layer 118 can be screen printed to the sensor. The passivation layer 118 can be a dielectric paste to passivate a portion of the electrodes when the sensor is placed in solution. The portion of the electrodes without the passivation layer provides a detection surface. The detection surface allows for the electrodes to be exposed to the solution when the sensor is placed in the solution and the chemical reaction of the sensing mechanism to take place. In some embodiments, the length 122 of the exposed electrodes can be about two centimeters.

At 108, the passivation layer 118 can be cured to provide a dielectric film. In some embodiments, the passivation layer 118 can be cured at a temperature of 120 degrees Celsius for 60 minutes. Curing the passivation layer 188 can also remove solvent.

Reference now is made to FIG. 1B, which illustrates an example setup 130 for obtaining a measurement using a potentiometric sensor, in accordance with at least one embodiment. As shown in FIG. 1B, the potentiometric sensor can include a working electrode 112, a counter electrode 114, and a reference electrode 122. For example, the working electrode 112 can be cobalt, the counter electrode (i.e., auxiliary electrode) 114 can be stainless-steel, and the reference electrode 122 can be a double junction silver-silver chloride (Ag/AgCl).

The potentiometric sensor can be placed in a water sample 132. A source measure unit 134 can be connected to each electrode of the potentiometric sensor. In some embodiments, the source measure unit can be operated in a potentiostat mode.

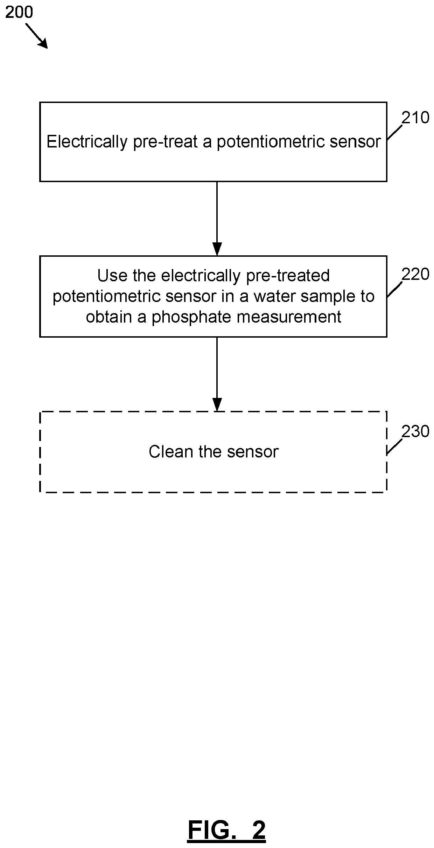

Reference now is made to FIG. 2, which illustrates a flowchart for a method 200 for measuring phosphate in water, in accordance with at least one embodiment. To assist with the description of the method 200 shown in FIG. 2, reference will be made simultaneously to FIGS. 1A and 1B.

Method 200 can begin at 210, when a potentiometric sensor, such as a sensor fabricated from process 100 of FIG. 1A, is electrically pre-treated. The potentiometric sensor can include a working electrode and a counter electrode. The counter electrode can be a stainless-steel electrode. The working electrode can include a metal/metal oxide electrode having a detection surface. For example, the detection surface can be a portion of the metal/metal oxide electrode that is exposed to the water without a passivation layer. As shown in FIG. 1A, the metal/metal oxide electrode can be a cobalt/cobalt-oxide electrode. In other embodiments, the metal/metal oxide electrode can be a molybdenum/molybdenum oxide electrode or a tungsten/tungsten oxide electrode.

In some embodiments, the sensor can also include a reference electrode. For example, as shown in FIG. 1B, the reference electrode can be a silver-silver chloride (Ag/AgCl) electrode.

Electrically pre-treating the potentiometric sensor can involve applying an anodic current between the working electrode and the counter electrode. The anodic current can have a density between about -1 mA/cm$^2$ to about -20 mA/cm$^2$. In at least one embodiment, the anodic current can have a density of about -9.18 mA/cm$^2$ (-1.43 $\mu$A).

The electrical pre-treatment can be performed in situ in a water sample. That is, the electrical pre-treatment can be performed on the sensor in the water sample that is being measured. This can allow for the electrical pre-treatment to be performed in the field - such as in bodies of water.

The electrical pre-treatment promotes oxidation of the metal/metal oxide electrode. The electrical pre-treatment increases anodic oxidation and results in the formation of a more stable film of mixed oxide during the measurement. The electrical pre-treatment can be applied until metal phosphate is formed on the detection surface of the metal/metal oxide electrode. In the case of a cobalt/cobalt oxide working electrode, the electrical pre-treatment can result in the formation of a cobalt phosphate film. In the case of a molybdenum/molybdenum oxide working electrode, the electrical pre-treatment can result in the formation of a molybdenum phosphate film. In the case of a tungsten/tungsten oxide working electrode, the electrical pre-treatment can result in the formation of a tungsten phosphate film.

To apply the electrical pre-treatment until at least some metal phosphate is generated on the detection surface, the anodic current can be a pulse having pre-determined pulse duration. For example, the pre-determined pulse duration can be between 150 seconds to 250 seconds. In at least one embodiment, the pre-determined pulse duration can be about 220 seconds.

At 220, the electrically pre-treated potentiometric sensor can be used in the water sample to obtain a phosphate measurement. The phosphate measurement can be a phosphate concentration. In some embodiments, the potentiometric sensor can be operated in an open circuit mode for at least a pre-determined measurement duration for the sensing mechanism to take place. For example, the pre-determined measurement duration can be about 110 seconds.

The sensing mechanism results in a fresh layer of mixed oxide on the detection surface of the metal/metal oxide electrode. The mixed oxide can include metal hydroxides, metal oxy hydroxides, metal oxides of various valencies, and any combination thereof.

As indicated by dashed lines, in some embodiments, the method 200 can also involve cleaning the sensor at 230, after obtaining the phosphate measurement. The sensor can be cleaned by removing mixed oxide from the detection surface to regenerate the detection surface. In some embodiments, cleaning the sensor can involve chemically dissolving mixed oxide from the detection surface.

In some embodiments, cleaning the sensor can involve using sandpaper to mechanically remove mixed oxide from the detection surface. For example, sandpaper can be used to polish the detection surface. The sandpaper can be a silicon carbide sandpaper. For example, the sandpaper can be of grade 600.

As described above, the sensor includes a passivation layer that covers a respective portion of the electrodes. The portion of the electrodes without the passivation layer provide a detection surface at which the sensing mechanism can take place. In some embodiments, cleaning the sensor can involve removing at least a portion of a passivation layer on the metal/metal oxide electrode to increase the detection surface. In some embodiments, both chemical and mechanical cleaning methods can be used.

In some embodiments, the method 200 can also involve cleaning the sensor prior to electrically pre-treating the sensor at 210 (not shown in FIG. 2). Cleaning the sensor prior to electrically pre-treating the sensor can remove hydroxide film that may have formed due to air exposure, as well as any residual mixed oxide.

In some embodiments, the method 200 can involve cleaning the detection surface both prior to electrically pre-treating the sensor at 210 and after obtaining the phosphate measurement at 220.

In some embodiments, the method 200 can involve obtaining a dissolved oxygen measurement of the water (not shown in FIG. 2) after obtaining the phosphate measurement at 220.

Method 200 can eliminate the need for chemical pre-conditioning of potentiometric sensors. By avoid the need for reagents when obtaining a measurement, method 200 allows cobalt-based sensors to be used in field measurements of water bodies. Method 200 can also decrease the time required for pre-conditioning. Furthermore, method 200 can also improve the sensitivity of the potentiometric sensors compared to existing methods.

Experiments were conducted to demonstrate the method 200. The experiments were carried out using a sensor fabricated via the example process 100 of FIG. 1A and the example set up 130 of FIG. 1B. Similar to FIG. 1B, the sensor was placed in a beaker with 100 mL of a sample solution. That is, the measurements were performed in dip format. To simulate fresh water, which has a salinity of less than 1000 ppm, potassium dihydrogen phosphate ($KH_2PO_4$) in an inert electrolyte was used as the sample solution. For example, 20 mM (1170 ppm) sodium chloride (NaCl) was used as the inert electrolyte. The pH, and conductivity of the sodium chloride (NaCl) solution was 6, and 2.26 mS respectively.

With cobalt-based sensors, the metallic cobalt is covered with a passive film of cobalt oxide (CoO) and hydroxide ($Co(OH)_2$), like other transition metals. When exposed to water, the water molecules can adsorb on the metal surface (Reaction 1 and 2) and result in the formation of multiple cobalt oxides/hydroxides such as CoO, $Co(OH)_2$ and $Co_3O_4$. The composition of this film depends strongly on the pH of the solution and the potential applied.

$$Co + H_2O \leftrightarrow Co(H_2O)_{ads} \qquad (1)$$

$$Co(H_2O)_{ads} \rightarrow CoOH^+ + H^+ + e^- \qquad (2)$$

In acidic solutions, the intermediate hydroxide generates cobalt ($Co^{2+}$) ion (Reaction 3) and continues the dissolution of cobalt metal.

$$CoOH^+ + H^+ \rightarrow Co^{2+} + H_2O \qquad (3)$$

In neutral solutions, the intermediate hydroxide generates $Co(OH)_2$ and CoO (Reaction 4-5), which eventually stabilizes the passivation film.

$$CoOH^+ + H_2O \rightarrow Co(OH)_2 + H^+ \qquad (4)$$

$$Co(OH)_2 \rightarrow CoO + H_2O \qquad (5)$$

In basic solutions, the film is further stabilized with a secondary film of mixed cobalt oxide ($Co_3O_4$) and cobalt oxyhydroxide (CoOOH).

$$CoOH^+ + OH^- \rightarrow Co(OH)_2 \qquad (6)$$

$$Co(OH)_2 + OH^- \rightarrow CoOOH + H_2O + 2e^- \qquad (7)$$

$$3CoO + 2OH^- \rightarrow Co_3O_4 + H_2O + 2e^- \qquad (8)$$

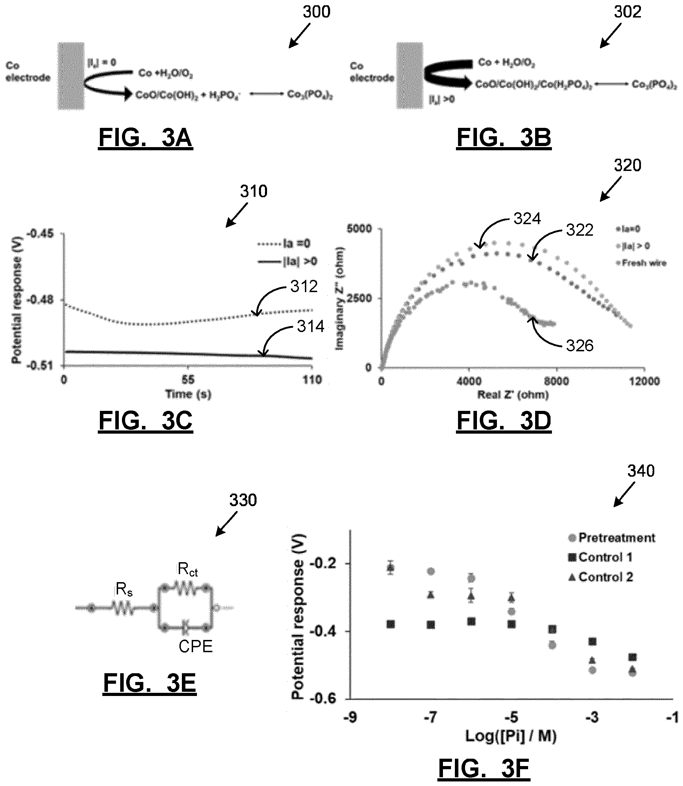

Reference is now made to FIG. 3A, which illustrates the sensing mechanism of a cobalt-based sensor, and in particular, the reaction 300 of the cobalt/cobalt-oxide working electrode without electrical pre-treatment, and FIG. 3B, which illustrates the reaction 302 of the cobalt/cobalt-oxide working electrode with electrical pre-treatment, in accordance with at least one embodiment. As can be seen, the electrical pre-treatment process can promote the oxidation of metallic cobalt.

Reference is now made to FIG. 3C, which compares the stability of the cobalt/cobalt-oxide working electrode potential over time 310, when the sensor is exposed to $10^{-3}$ M $KH_2PO_4$ without electrical pre-treatment 312 and with electrical pre-treatment 314, in accordance with at least one embodiment. The electrode with electrical pre-treatment 314 exhibits a more negative potential shift due to the formation of a more stable film of cobalt oxide compared to that of the electrode without electrical pre-treatment 312. The more stable film of cobalt oxide is a result of enhanced or increased anodic oxidation of metallic cobalt to various oxides with electrical pre-treatment.

The cobalt electrode was electrically pre-treated in situ in the presence of other ions like sodium, chloride, dihydrogen phosphate, and potassium. These other ions can adsorb on the passivation film during the electrical pre-treatment and measurement. Thus, these ions were observed on the sensor surface in the survey spectra using X-ray photoelectron spectroscopy (XPS) measurement. Moreover, dihydrogen phosphate is known to form complex under applied potential. Therefore, another reaction can occur in the presence of dihydrogen phosphate during electrical pretreatment (Reaction 9).

$$Co(H_2PO_4)_2 + 2e^- \rightarrow Co + 2H_2PO_4^- \qquad (9)$$

Finally, the oxide/hydroxide/phosphate films convert to cobalt (II) phosphate. Based on the XPS results, the following reactions are expected to occur at the interface to generate cobalt (II) phosphate (Reaction 10, 11, and 12):

$$2Co_3O_4 + 3Co(H_2PO_4)_2 \leftrightarrow 3\,CO_3(PO_4)_2 + 6H_2O + O_2 \qquad (10)$$

$$2CoO + Co(H_2PO_4)_2 \leftrightarrow Co_3(PO_4)_2 + 2H_2O \qquad (11)$$

$$2Co(OH)_2 + 3Co(H_2PO_4)_2 \leftrightarrow 3Co_3(PO_4)_2 + 4H_2O \qquad (12)$$

Electrochemical Impedance Spectroscopy (EIS) was performed to characterize the change in ionic and electronic charge transfer at the electrode-electrolyte interface with electrical pre-treatment. The impedance data at low frequency is related to the ionic charge transfer while the impedance data at high frequencies provides insight in interfacial electronic transfer. EIS measurements were performed in $10^{-3}$M $KH_2PO_4$ samples for sensors with and without the electrical pre-treatment.

Reference is now made to FIG. 3D, which is a Nyquist plot 320 of the electrochemical impedance measurements of an electrode after sensing (i.e., with phosphate exposure) without electrical pre-treatment 322, an electrode after sensing (i.e., with phosphate exposure) with electrical pre-treatment 324 in accordance with at least one embodiment, and an electrode prior to sensing (i.e., without phosphate exposure) and without electrical pre-treatment 326.

A Randles circuit 330 with three components, shown in FIG. 3E, was fitted to the electrochemical impedance measurements of FIG. 3D. The three circuit components included a solution resistance ($R_s$), a charge transfer resistance ($R_{ct}$), and a constant phase element (CPE) representing a combined effect of double layer and cobalt phosphate film on the electrode. The CPE was used instead of an ideal capacitive element, to capture the heterogeneity of the electrode surface. The impedance of CPE is defined as:

$$Z(CPE) = [Q(j\omega)^n]^{-1} \qquad (13)$$

where

Q is a constant;

n is CPE power; and

$\omega$ is angular frequency.

All fitted data had a $\chi^2$ value in the order of $10^{-3}$ and the error associated with all of the components were <3.2%. No significant changes were observed in $R_s$ for all the three cases 322, 324, and 326. A significant increase in diameter of the semicircle was recorded for the Nyquist plot when the phosphate measurement was performed with electrical pre-treatment as compared to without electrical pre-treatment. An increase in both the Q value for CPE (21%) and $R_{ct}$ (6%) were observed with pretreatment as compared to without pretreatment when the data was fitted to the electrical circuit. The increase in the $R_{ct}$ and CPE for electrically pre-treated sensor can be attributed to a thicker insoluble cobalt oxide/phosphate film on the sensor resulting in the decreased electrochemical activity of cobalt oxide film.

XPS was used to further investigate the changes in composition of cobalt sensor surface before and after electrical pre-treatment and after measurement. Three cobalt sensors were prepared and exposed to $10^{-2}$ M $KH_2PO_4$. A first sample (Sample 1) was a sensor without electrical pre-treatment that had been exposed to $10^{-2}$ M $KH_2PO_4$ during potentiometric measurement. A second sample (Sample 2) was a sensor with electrical pre-treatment that had been exposed to $10^{-2}$ M $KH_2PO_4$. A third sample (Sample 3) was a sensor with electrical pre-treatment followed by zero current potentiometric measurement in $10^{-2}$ M $KH_2PO_4$.

The spectra showed that the sensor surface was composed of cobalt, phosphorus, oxygen, potassium, sodium, chlorine, along with some traces of nitrogen and silicon. The presence of cobalt, phosphorus, oxygen, potassium, sodium and chlorine was expected and arose from the sensor itself or the solutions in which they are dipped in. The traces of nitrogen and silicon can be a result of the preparatory procedures as the sensors were sealed in nitrogen after the experiment to avoid contact with oxygen prior to XPS analysis and the sensor was first polished using silicon sandpaper prior to the electrical pre-treatment.

High resolution spectra of cobalt (Co 2p) was obtained from the sensor surfaces to determine the distribution of specific cobalt species present on the surface. The spectra was fitted mainly with five cobalt species: metallic cobalt (Co(0)), cobalt (II) hydroxide ($CO(OH)_2$), cobalt phosphate ($Co_3(PO_4)_2$), cobalt (II, III) oxide ($Co_3O_4$) and cobalt oxyhydroxide (CoOOH). The distribution of the cobalt species for three samples from the high-resolution spectra is summarized in Table 1. The numbers in Table 1 represent the atomic percentage of different forms of cobalt. Due to a significant overlap in the peaks of $Co(OH)_2$ and $Co_3PO_4$, the percentage of $Co_3PO_4$ was estimated from the total cobalt-based on the stoichiometry from the survey scan results.

**Table 1**

| Sample Identifier | Co(0) | Co(OH)₂* | Co₃(PO4)₂* | Co₃O₄ | CoOOH |
|---|---|---|---|---|---|
| Sample 1 | 2 | 46 | 41 | 0 | 12 |
| Sample 2 | 5 | 21 | 65 | 0 | 9 |
| Sample 3 | 10 | 29 | 40 | 13 | 8 |

A significant increase in $Co_3O_4$ was observed for Sample 3 (13%) compared to Sample 1 (0%) and Sample 2 (0%). As well, a higher amount of $Co_3(PO_4)_2$ was present on the sensor surface for Sample 2 (65%) compared to Sample 1 (41%), which may be a result of the presence of $NaH_2PO_4$ due to incorporation of insoluble cobalt phosphate in the film. However, this increase in cobalt phosphate was reduced for Sample 3 (40%), but an increase in the ratio of Co3+/Co2+ was observed as compared to Sample 1.

The effect of the electrical pre-treatment on the sensing performance of the cobalt/cobalt oxide sensor was evaluated. The sensors prepared were placed in sample solution (standards) with phosphate concentration between $10^{-8}$ M to $10^{-2}$ M. The pH of the standard phosphate solutions from $10^{-8}$ M to $10^{-3}$ M was between 5.9 - 6.2. The pH for $10^{-2}$ M was 5.2.

Reference is now made to FIG. 3F, which compares the effect of the electrical pre-treatment on the potential response of the sensor 340, in accordance with at least one embodiment, compared to control experiments without electrical pre-treatment of the sensor. The potential response of a sensor that was electrically pre-treated, immediately followed by open circuit potential measurement in 20 mM sodium chloride (NaCl) solution as the inert electrolyte is shown (circles). The control experiments were performed to investigate the effect of the inert electrolyte and the pH on the sensitivity of the sensor. In a first control experiment (Control 1 - shown in rectangles), a sensor was not electrically pre-treated and 25 mM potassium hydrogen phthalate (KHP) buffer solution was used as the inert electrolyte. KHP buffer solution is commonly used as the base electrolyte in conventional methods of measuring phosphate using cobalt-based sensors. In a second control experiment (Control 2 - shown in triangles), a sensor was not electrically pre-treated and

sodium chloride (NaCl) was also used as the inert electrolyte.

As can be seen in FIG. 3F, in addition to alleviating the need for chemical pre-conditioning, electrical pre-treatment also increases the sensitivity of the sensor. With electrical pre-treatment (shown in circles), the sensors exhibited a linear range from $10^{-6}$ to $10^{-3}$ M with increased sensitivity of -91.4 mV/ decade of phosphate concentration.

In Control 1, the sensor had a linear range from $10^{-5}$ to $10^{-1}$ M with a sensitivity of only -36.5 mV/ decade of phosphate concentration (shown in rectangles). Comparison of the measurements obtained from the sensor with electrical pre-treatment and Control 1 confirms that the increased sensitivity is not due to the change from 25 mM KHP buffer (i.e., the base electrolyte of conventional methods of measuring phosphate) to 20 mM NaCl solution.

In Control 2, the sensors exhibited a linear range from $10^{-5}$ to $10^{-2}$ M with a sensitivity of -72.4 mV/ decade of phosphate concentration (shown in triangles). Comparison of the measurements obtained from the sensor with electrical pre-treatment and Control 2 demonstrates that the electrical pre-treatment can increase the sensor sensitivity and extend the lower range of the sensor.

The anodic current applied during the electrical pre-treatment step can promote water molecules adsorbing on the metal surface, resulting in the formation of additional cobalt oxide (Reaction 1). This was observed in with the drop in the potential response shown in FIG. 3F to more negative values within $10^{-5}$ M to $10^{-3}$ M with electrical pre-treatment compared to the potential responses without electrical pre-treatment.

In addition, the electrical pre-treatment resulted in an early saturation of the potential response at $10^{-3}$ M while the potential response saturated at $10^{-2}$ M in Control 2 and $10^{-1}$ M in Control 1 (not shown in FIG. 3F). The potential response observed in FIG. 3F is consistent with the Pourbaix diagram for $Co-H_2O$ at 25 degrees Celsius, which indicates that cobalt does not oxidize at negative potentials greater than -0.5 V at pH < 9. The limit at one end of the linear range for the sensor with electrical pre-treatment was - 0.514 V ($10^{-3}$ M phosphate solution), -0.519 V in Control 1 ($10^{-1}$ M phosphate solution, not shown in FIG. 3F), and -0.511 V in Control 2 ($10^{-2}$ M phosphate solution). The limit at the other end of the linear range for the sensor with electrical pre-treatment was -0.340 V, -0.378 V in Control 1 ($10^{-5}$ M phosphate solution), and -0.340 V in Control 2. The lower potential response of Control 1 may be due to more cobalt oxidation at pH 4 (25 mM KHP buffer solution) as compared to pH 6 (20mM NaCl solution with electrical pre-treatment and Control 2).

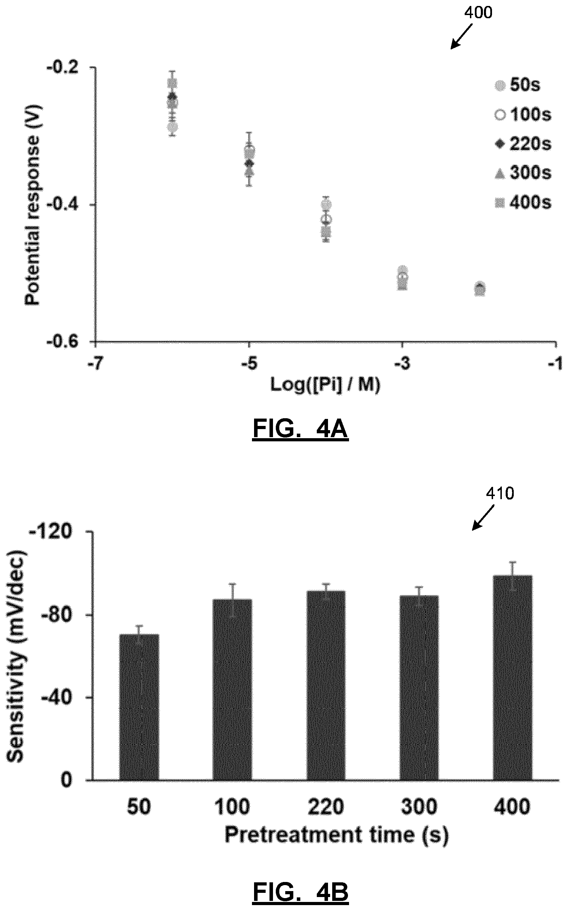

Reference is now made to FIG. 4A, which compares the effect of the time duration of the electrical pre-treatment on the potential response of the sensor 400, in accordance with at least one embodiment. FIG. 4A shows the potential response of sensors with five different electrical pre-treatment time durations: 50 seconds (shown in filled circles), 100 seconds (shown in open circles), 220 seconds (shown in diamonds), 300 seconds (shown in triangles) and 400 seconds (shown in rectangles). An anodic current of -1.43 $\mu$A was applied in all five time durations. The potential responses were calculated as the average of the last 10 seconds of the potentiometric measurement. As shown in FIG. 4A, the electrically pre-treated electrodes respond linearly to a range of phosphate concentrations between 10-6 M to 10-3 M in the sample.

Reference is now made to FIG. 4B, which compares the effect of the time duration of the electrical pre-treatment on the sensitivity of the sensor 410, in accordance with at least one embodiment. The sensitivity of the sensors is determined based on the slope of the calibration curves.

When the electrical pre-treatment was applied for a short duration of time (e.g., 50 seconds), the slope of the calibration curve was relatively low at -70.3 mV/decade with a $R^2$ value of 0.955. This slope is similar in magnitude to that obtained when fresh Co wire was used as an electrode, without electrical pre-treatment, which indicates that the shorter time duration was not sufficient for formation of the cobalt phosphate film that improves sensitivity. With an increase in the electrical pre-treatment time duration, the sensitivity increases to -86.83 mV/decade at 100 seconds, -91.4 mV/decade at 220 seconds, and -88.9 mV/decade at 300 seconds. The variation in the sensor response was also higher for shorter electrical pre-treatment durations, which can indicate that the electrical pre-treatment did not reach an equilibrium state. As shown in FIG. 4B, an electrical pre-treatment time duration of 220 seconds can be optimal for enhanced sensitivity.

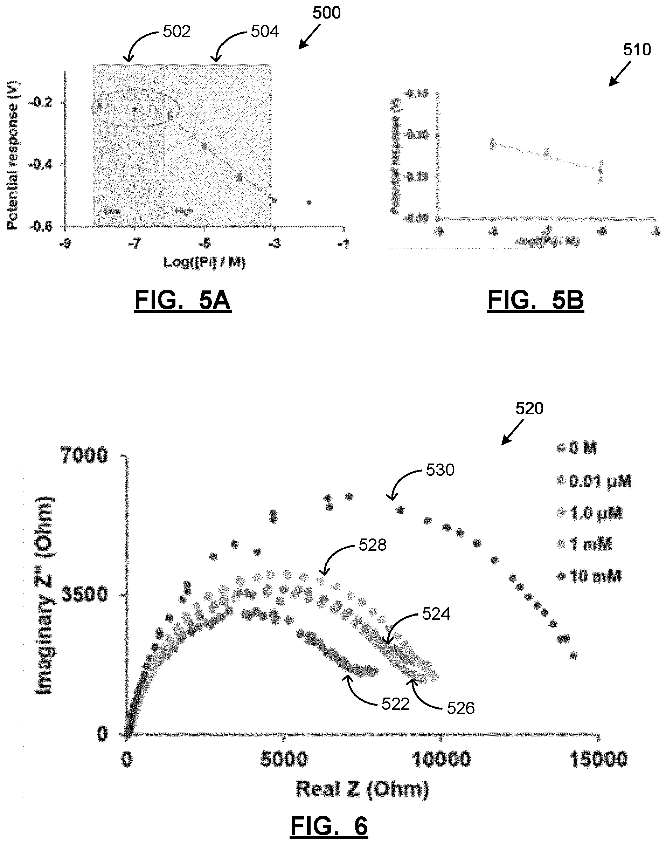

Reference is now made to FIG. 5A and 5B, which compares the dynamic response of the sensor in different standard phosphate solutions 500, in accordance with at least one embodiment. In particular, the sensor was tested in seven different solutions with phosphate concentration ranging from $10^{-2}$ M to $10^{-8}$ M. The solutions were prepared by dissolving potassium dihydrogen phosphate in 20 mM NaCl.

Solutions having a phosphate concentration between from $10^{-3}$ M to $10^{-6}$ M can be a main testing region 504 while solutions having a phosphate concentration between $10^{-6}$ M to $10^{-8}$ M can be a lower concentration testing region 502. FIG. 5B shows a magnified view 510 of the dynamic response of the sensor in the lower concentration testing region 502. The dynamic response of the sensor in the main testing region 504 can be used to define the working range of the sensor in a solution matrix.

The slope of the curve shown in FIG. 5A is -91.4 mV/decade of phosphate concentration change between $10^{-3}$ M to $10^{-6}$ M with a $R^2$ value of 0.9959, indicating a good linear fit for the sensor response. The high sensitivity can be due to the presence of mixed oxide on the surface of the electrically pre-treated sensor.

As shown in Table 1 above, an increased amount of cobalt phosphate was observed on the sensor surface just after the electrical pre-treatment. The increased amount of cobalt phosphate can indicate higher phosphate incorporation in the surface film than in the sensor without electrical pre-treatment. During the measurement step after electrical pre-treatment, the amount of $Co_3O_4$ on the sensor surface increased from 0% for the sensors without electrical pre-treatment to 13% for electrically pre-treated sensors. This increase confirms that the electrical pre-treatment enhances the ratio of Co3+/Co2+ present on the surface on the oxide/phosphate film. The change in oxidation state of the film resulted in a mixed oxide and non-Nernstian response observed for the electrically pre-treated electrodes.

The high sensitivity of the electrically pre-treated sensor is similar to the high sensitivity of pH sensors fabricated using electrodeposited iridium oxide film. Similarly, the high sensitivity (i.e., non-Nernstain response) of such pH sensors is due to the presence of multiple oxidation states of iridium ($Ir^{3+}$ and $Ir^{4+}$) in the oxide film and the mixed potential response due to the non-redox reactions. Other non-redox reactions can also contribute to the high pH sensitivity.

The sensitivity of the electrically pre-treated sensor is higher than the sensitivity of several conventional sensors, including but not limited to, cobalt-based wire sensors, which have a sensitivity of -19 to -54 mV/decade, molybdenum based electrodes, which have a sensitivity of -26.9 to -27.8 mV/decade, and ion selective membrane based sensors, which have a sensitivity of -28 to -55.7 mV/decade. The higher sensitivity of electrically pre-treated sensor can be attributed to the anodic current pre-treatment, which favors the conversion of Co to Co2+.

Returning to FIG. 5A, the sensor response was saturated beyond a concentration of $10^{-3}$ M. This can be understood from the Pourbaix diagram for Cobalt at 25 degrees Celsius, which indicates a saturation of oxidation potential at about -0.5 V under current conditions. Therefore, no further potential changes can occur, resulting in a constant sensor response with increased phosphate concentration. The electrically pre-treated sensor can saturate for concentrations >1 mM of dihydrogen phosphate. At 1 mM, the surface of the electrically pre-treated sensor can lack of active cobalt, which prevents the formation of additional cobalt oxide with phosphate concentrations beyond 1 mM.

As shown in FIG. 5B, the electrical pre-treatment can extend the lower detection range to a phosphate concentration of approximately $10^{-8}$ M. In comparison, most cobalt-based sensors only work within a wide range from $10^{-5}$ M to $10^{-2}$ M. Some cobalt-based sensors may have a wider range, such as $10^{-6}$ M to $10^{-1}$ M, but only within a pH of between 3.75 to 4, which may not be suitable for most phosphate measurement applications. Similarly, molybdenum base sensors may work within a range of $10^{-5}$ to $10^{-2}$ M is limited to alkaline pH levels.

Reference is now made to FIG. 6, which is a Nyquist plot 520 of the electrochemical impedance measurements for different standard concentrations, in accordance with at least one embodiment. In particular, FIG. 6 shows a Nyquist plot for a sensor unexposed to phosphate 522 and electrically pre-treated sensors exposed to four different $KH_2PO_4$ concentrations: 0.01 μM (shown as 524), 1 μM (shown as 526), 1 mM (shown as 528) and 10 mM (shown as 530).

Electrochemical impedance measurements are obtained using EIS to understand changes at the electrode-electrolyte interface with electrical pre-treatment and the effect of different phosphate concentration on the electrode-electrolyte interface. The results were fitted to a Randles circuit, as shown in FIG. 3E.

$R_s$ changes depending on the spiked $KH_2PO_4$ concentrations. For example, a resistance change of about 8% was observed for 0.01 μM $KH_2PO_4$, about 16% for 10mM $KH_2PO_4$.

When the sensor was exposed to the 0.01 μM $KH_2PO_4$ concentration (shown as 524), a 26% increase in $R_{ct}$ was observed as compared to a sensor with no phosphate exposure 522. The increase in $R_{ct}$ can be linked to the formation of the cobalt phosphate film on the surface with phosphate exposure. Small increases of 28% and 31% in $R_{ct}$ was observed for 1 μM $KH_2PO_4$ concentration (shown as 526) and 1 mM $KH_2PO_4$ concentration (shown as 528), respectively. The small increase in $R_{ct}$ can be related to formation of additional cobalt phosphate film. However, this increase in $R_{ct}$ did not have a significant impact on the charge transfer on the electrode-electrolyte interface. A 93% increase in $R_{ct}$ was observed for 10 mM $KH_2PO_4$ concentration shown as 530), showing the formation of a thicker phosphate film, which was further confirmed by the sensor response saturation at 10 mM $KH_2PO_4$. No significant changes were observed for the Q value of CPE.

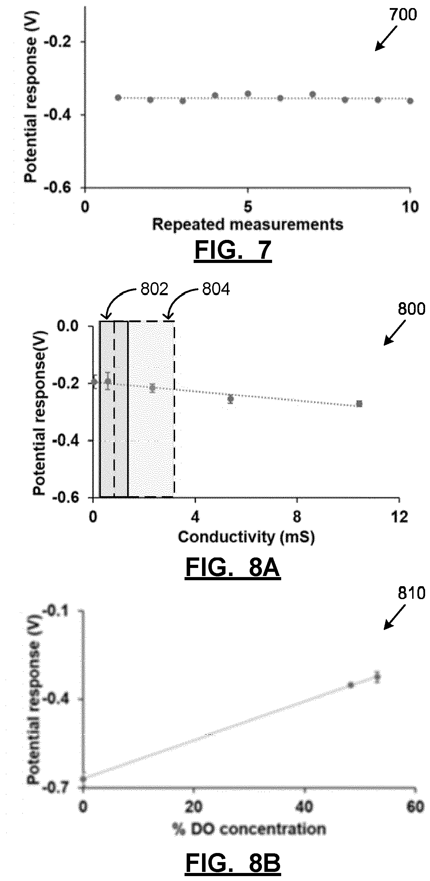

Reference is now made to FIG. 7, which illustrates repeatability data 700 for the electrically pre-treated sensor, in accordance with at least one embodiment. The repeatability data 700 was obtained by repeatedly electrically pre-treating the sensor, using the sensor to obtain measurements, and polishing the sensor. The sensor was tested in $10^{-5}$ M potassium dihydrogen solution.

As shown in FIG. 7, an average potential response of -0.353 V with a standard deviation of 0.7% after 10 measurements. The relative standard deviation (R.S.D) for the 10 measurements was 2.1%, which was comparable to the existing sensors. For example, the R.S.D. can be 0.5% for screen printed cobalt-based sensors, 3.8% and 3% for bulk cobalt wire, and 0.6% for on-chip phosphate sensors.

A comparison of a polished and an unpolished cobalt electrode surface was conducted with surface morphology using Scanning Electron Microscope (SEM) and energy dispersive X-ray spectroscopy (EDX). The surface morphology confirmed the absence of oxygen on the polished cobalt electrode surface, which indicates that polishing can effectively regenerate the sensor surface.

The sensor-to-sensor reproducibility was also demonstrated by fabricating four sensors and testing the sensor with $10^{-5}$ M potassium dihydrogen solution. The average sensor response was -340.14 mV with a standard deviation of 0.8% and R.S.D of 2%. The relative standard deviation (R.S.D) is comparable to the existing sensors, which can be between about 1.9% to 3.2% for screen printed cobalt-based sensors, and 2.5% for on-chip phosphate sensors.

The electrode response for cobalt-based sensor is a mixed potential response. The sensor response depends on factors like conductivity, dissolved oxygen, and pH levels. The sensor was also tested with common interfering anions present in surface water like nitrates, sulphate and chlorides.

Reference is now made to FIG. 8A, which compares the effect of different conductivities 800, in accordance with at least one embodiment. In particular, the sensor was tested in five different solutions having conductivities of 0.05 mS, 0.6 mS, 2.3 mS, 5.3 mS and 10.4 mS. The solutions were prepared by dissolving sodium chloride in deionized water. Each point in FIG. 8A represents the average of multiple sensors and the error bar represents the standard deviation of the response of multiple sensors. The slope of the calibration curve was -8.2 mV/mS with an y-intercept of -0.195 V and $R^2$ value of 0.9254.

The conductivity of the water varies depending on the source of water. As shown in FIG. 8A, the conductivity of potable water 802 can range from 0.030 mS to 1.5 mS while the conductivity of freshwater streams 804 can range from 0.1 mS to 2 mS. For potable water, the slope of the calibration curve indicates that a change in conductivity from about 0.5 mS to 1 mS will result in a 4.1 mV change, which is 0.045 times of the sensitivity per decade of phosphate measurements.

Reference is now made to FIG. 8B, which compares the effect of different dissolved oxygen concentrations 810, in accordance with at least one embodiment. In particular, the sensor was exposed to three different solutions having dissolved oxygen concentrations of 0%, 48.3% and 53.1%. The solutions were prepared by dissolving oxygen in 10 $\mu$M of $KH_2PO_4$. Each point in FIG. 8B represents the average of multiple sensors and the error bar represents the standard deviation of the response of multiple sensors.

As shown in FIG. 8B, the sensor response is positively related to the oxygen concentration, which is unlike phosphate where the potential decreases with an increase in phosphate concentration. Also shown in FIG. 8B, a change of 65 mV per 10% change in dissolved oxygen change was observed. This is a significant change as compared to the sensitivity of the sensor.

The potential change is comparable to some conventional cobalt wire-based sensors where dissolved oxygen change from 0% to 21 % can result in a potential change from -0.58V to -0.33V in $10^{-4}$ M standard phosphate solution at pH 7.5. Therefore, similar to conventional cobalt-based sensors, a dissolved oxygen measurement should be performed with when a measurement is obtained with the electrically pre-treated sensor.

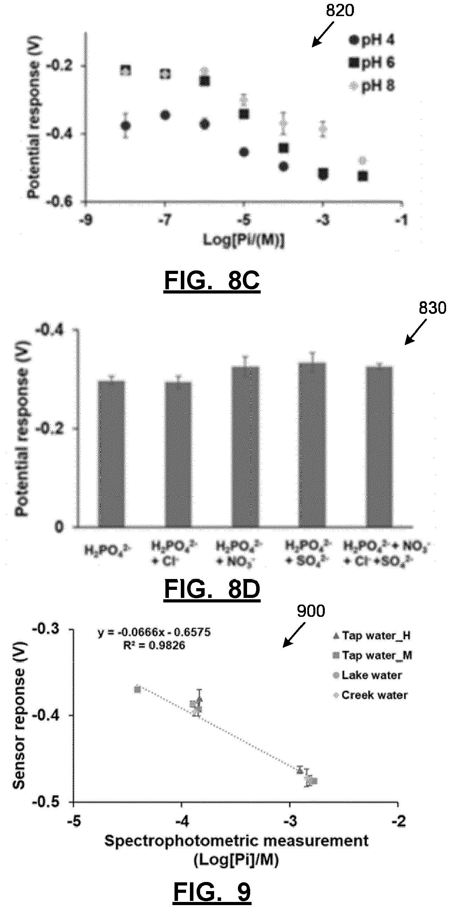

Reference is now made to FIG. 8C, which compares the effect of different pH levels 820, in accordance with at least one embodiment. In particular, the sensor was tested in three different solutions having pH of 4, 6, 8, and 10. The solutions were prepared using 20mM NaCl as the inert electrolyte. The pH was adjusted using potassium hydroxide and hydrochloric acid. The pH level can change the composition of the phosphate species present in the analyte solution and the sensor response can depend on pH of the solution.

As shown in FIG. 8C, the sensor response at pH level 4 exhibited a calibration slope of -48.1 mV/decade ($R^2$ value = 0.9639) for a linear range from $10^{-7}$ M to $10^{-3}$ M (shown as circles). The calibration slope was similar to that of FIG. 5A in which the electrical pre-treatment showed an extension of the measuring range to $10^{-7}$ M. At pH 4, the potential response for $10^{-3}$ M showed a higher negative potential (-0.332 V) compared to pH 6 (-0.222 V) and pH 8 (-0.225 V). The higher negative potential at pH 4 can be due to more cobalt oxidation as compared to neutral (pH 6) and alkaline (pH 8) solutions.

The measuring range of sensor at pH 8 was from $10^{-6}$ to $10^{-2}$ M ($R^2$ value = 0.965) with a calibration slope of -61.3 mV (shown as diamonds). The decrease in slope can be due to the beginning of inactivation of the cobalt surface due to the alkaline pH. The sensor failed to perform at pH 10 (shown in FIG. 8C).

Since the range of pH values in typical environmental samples is typically small, the electrical pre-treatment can be used for samples with smaller pH variations (about $\pm$0.5 pH changes). For larger pH variations, the sensor's response can be normalized with pH values.

Reference is now made to FIG. 8D, which compares the effect of common interferents on the sensor response 830, in accordance with at least one embodiment. In particular, the sensor was tested for three common coexisting anions present in tap water and surface water: chloride ($Cl^-$), nitrates ($NO_3$) and sulphates ($SO_4^{2-}$). Each bar plot in FIG. 8D represents the average value of the sensor response from multiple sensors and the error bar represents the standard deviation of the response of multiple sensors.

The sensors were electrically pre-treated, followed by measurement of a $KH_2PO_4$ solution, followed by a $KH_2PO_4$ solution with an interferent. The solution with interferents were prepared using sodium salt of chloride, nitrates, or sulphates. With the concentration of surface water nitrates being 0 to 18 mg/l, sulphates being 200 mg/l, and chloride being less than 250 mg/l, the measurements of FIG. 8D were obtained from a solution having a concentration of 50 mg/l of nitrate, 500 mg/l of sulphate and 500 mg/l of chloride dissolved in 10 $\mu$M of $KH_2PO_4$ sample solution.

As shown in FIG. 8D, minimal change was observed in the potential response of the sensor with the presence of coexisting anions. The selectivity coefficients calculated for chloride, nitrate and sulphate were -4.04, -1.75 and -3.58, respectively. These measurements are comparable to selectivity coefficients calculated from conventional sensors measurements. For example, the selectivity coefficient calculated from conventional sensors measurements is in the range of -2.3 to -4.0 for chloride; -3.1 to - 3.3 for nitrate; and -3.0 to -3.2 for sulphate. Therefore, the electrical pre-treatment does not have a significant effect on the sensor response in the presence of coexisting anions as compared to the chemical pre-conditioning in standard solutions. Hence, electrical pre-treatment can be used for pre-conditioning the sensor in situ before phosphate measurement.

Reference is now made to FIG. 9, which compares the sensor response to different water samples, in accordance with at least one embodiment. In particular, the sensor was electrically pre-treatment in situ and then used to measure phosphate in tap water (e.g., "Tap water_H" and "Tap water_M" shown in FIG. 9) and surface water (e.g., "Lake water" and "Creek water" shown in FIG. 9) samples. Each data point in FIG. 9 represents the average of multiple sensor data and the error bar represents the standard deviation of the response of multiple sensors.

Tap water samples were used as collected from the source. Surface water samples were filtered to remove the solid particles before measurement. For example, a 0.2 $\mu$m Whatman filter paper can be used to filter solid particles. Each of the samples were spiked with $KH_2PO_4$ to generate two different phosphate concentrations (1 mM and 0.1 mM).

Initially, a calibration curve was generated using the "Tap water_M" sample. The Tap water_M sample was sequentially spiked with three $KH_2PO_4$ concentrations ($10^{-5}$ M, $10^{-4}$ M, and $10^{-3}$ M) moving from low to high concentrations within a sample volume of 100 mL. The final phosphate concentrations of the spiked samples were measured using a UV-Vis spectrophotometer with the Phosphomolybdic acid/ascorbic acid method. FIG. 9 illustrates the measured phosphate values were plotted against the potential response.

The three measured phosphate concentrations from standard spectrophotometric methods were $3.89\times10^{-5}$, $1.41\times10^{-4}$ and $1.66\times10^{-3}$ M, respectively. The sensor was able to differentiate between water samples spiked with the different $KH_2PO_4$ concentrations. The slope of the calibration curve (y (mV)=-66.6x [$KH_2PO_4$] -657.5) was -66.6 mV/decade of $KH_2PO_4$ concentrations in tap water. This calibration slope is 32% higher than the calibration slope of -50.6 mV/decade for a conventional cobalt-based sensor with chemical pre-conditioning and without electrical pre-treatment. However, the calibration slope is lower than the slope obtained in lab prepared samples where potassium dihydrogen phosphate was dissolved in in 20 mM NaCl standard solution, similar to Control 2. The lower calibration slope of the tap water samples can be linked to the change in pH, conductivity, and presence of interfering anions in the tap water samples. The pH of tap water_H and tap water_M were 7.19 and 7.47, respectively. The conductivity values were also less (0.33 mS for both tap water samples) as compared to the standard phosphate solution.

The sensor response was validated by spiking the lake water, tap water_H and creek water with two $KH_2PO_4$ concentrations each ($10^{-4}$ M and $10^{-3}$ M). The phosphate concentrations of the samples were measured using the standard phosphate method and the electrically pre-treated sensor, in accordance with at least one embodiment. The spectrophotometric measurements for the spiked $10^{-3}$ M lake water, tap water_H and creek water were $1.51 \times 10^{-3}$ M, $1.23 \times 10^{-3}$ M, and $1.41 \times 10^{-3}$ M, respectively. The predicted phosphate concentrations from the sensor response for the spiked $10^{-3}$ M lake water, tap water_H and creek water were $1.85 \pm 0.35 \times 10$-3 M, $1.20 \pm 0.17 \times 10$-3 M, and $1.73 \pm 0.64 \times 10$-3 M, respectively. Therefore, the phosphate recovery for the spiked $10^{-3}$ M lake water, tap water_H and creek water was 122%, 97% and 123%, respectively. A correlation plot between the phosphate concentration predicted from the sensor response and the spectrophotometric measurements showed a good correlation with a slope 1.068 and $R^2$ value of 0.9645. For lake water and creek water, the sensor response was similar to tap water samples for concentrations between $10^{-4}$ and $10^{-3}$ M. However, the sensor response was saturated between $10^{-5}$ and $10^{-4}$ M. This early sensor response saturation can be attributed to the presence of suspended solids, organic matter and chemicals in lake and creek water as compared to tap water. Further, pH of lake and creek water was 6.21 and 8.37, respectively, which can also contribute to the variation in sensor measurement.

The methods and apparatus disclosed herein can enhance the sensitivity of potentiometric sensors used for measuring phosphate in water, including cobalt-based sensors. In addition, the methods and apparatus alleviate the need for the long, chemical pre-conditioning that is conventionally required prior to measurement. Electrically pre-treating the potentiometric sensors can involve applying an anodic current to the sensor and can be performed in situ in the water sample itself prior to measurement. Furthermore, the electrical pre-treatment is relatively quick and can be performed in the field (i.e., not at a laboratory). No significant interference was detected with common interfering anions that are typically present in field water samples such as nitrate, sulphate and chloride. The methods and apparatus disclosed herein also demonstrated a good response to spiked tap water, lake water and creek water, illustrating the ability to perform phosphate measurement in real waters.

It will be appreciated that numerous specific details are set forth in order to provide a thorough understanding of the example embodiments described herein. However, it will be understood by those of ordinary skill in the art that the embodiments described herein may be practiced without these specific details. In other instances, well-known methods, procedures, and components have not been described in detail so as not to obscure the embodiments described herein. Furthermore, this description and the drawings are not to be considered as limiting the scope of the embodiments described herein in any way, but rather as merely describing the implementation of the various embodiments described herein.

It should be noted that terms of degree such as "substantially", "about" and "approximately" when used herein mean a reasonable amount of deviation of the modified term such that the end result is not significantly changed. These terms of degree should be construed as including a deviation of the modified term if this deviation would not negate the meaning of the term it modifies.

In addition, as used herein, the wording "and/or" is intended to represent an inclusive-or. That is, "X and/or Y" is intended to mean X or Y or both, for example. As a further example, "X, Y, and/or Z" is intended to mean X or Y or Z or any combination thereof.

It should be noted that the term "coupled" used herein indicates that two elements can be directly coupled to one another or coupled to one another through one or more intermediate elements.

Various embodiments have been described herein by way of example only. Various modification and variations may be made to these example embodiments without departing from the spirit and scope of the invention, which is limited only by the appended claims.

**Claims**

1. A method of measuring phosphate in water, the method comprising:

   - electrically pre-treating a potentiometric sensor, the potentiometric sensor comprising a working electrode and a counter electrode, the working electrode comprising a metal/metal oxide electrode having a detection surface, the electrical pre-treatment generating a metal phosphate on the detection surface of the metal/metal oxide electrode; and
   - using the electrically pre-treated potentiometric sensor in a water sample to obtain a phosphate measurement; wherein the phosphate measurement results in a fresh layer of mixed oxide on the detection surface of the metal/metal oxide electrode.

2. The method of claim 1, wherein the metal/metal oxide electrode comprises at least one selected from a group consisting of a cobalt/cobalt-oxide electrode, a molybdenum/molybdenum oxide electrode, and a tungsten/tungsten oxide electrode; and/or
   wherein the fresh layer of the mixed oxide comprises at least one selected from a group consisting of metal hydroxides, metal oxy hydroxides, and metal oxides.

3. The method of any one of claims 1 or 2 comprises electrically pre-treating the potentiometric sensor in situ in the water sample and/or an analyte solution.

4. The method of any one of claims 1 to 3 comprises applying an anodic current between the working electrode and the counter electrode;

   optionally wherein, the anodic current comprises a pulse having pre-determined pulse duration;
   further optionally wherein, the pre-determined pulse duration is about 220 seconds.

5. The method of any one of claims 1 to 4 wherein the anodic current has a density between about -1 mA/cm$^2$ to about - 20 mA/cm$^2$;
   optionally wherein the anodic current has a density of about -9.18 mA/cm$^2$.

6. The method of any one of claims 1 to 5 comprises using the potentiometric sensor in an open circuit mode for at least a pre-determined measurement duration to obtain the measurement;
   optionally wherein, the pre-determined measurement duration is about 110 seconds.

7. The method of any one of claims 1 to 6 further comprising removing mixed oxide from the detection surface prior to electrically pre-treating the sensor and/or after obtaining the phosphate measurement;

   optionally,

   chemically dissolving mixed oxide from the detection surface; and/or
   removing at least a portion of a passivation layer on the metal/metal oxide electrode to increase the detection surface; and/or
   using sandpaper to mechanically remove mixed oxide from the detection surface;

further optionally wherein, the sandpaper comprises silicon carbide.

8. The method of any one of claims 1 to 7, wherein the counter electrode comprises a stainless-steel electrode.

9. The method of any one of claims 1 to 8, wherein the potentiometric sensor further comprises a reference electrode; optionally wherein, the reference electrode comprises a double junction silver-silver chloride electrode.

10. The method of any one of claims 1 to 9 further comprising obtaining a dissolved oxygen concentration of the water.

11. An apparatus for field monitoring of water, the apparatus comprising:

- a potentiometric sensor operable to measure of phosphate in situ in a water sample, the potentiometric sensor comprising a working electrode and a counter electrode, the working electrode comprising a metal/metal oxide electrode; and
- an electrical source connectable to the working electrode and the counter electrode for applying an anodic current prior to measuring the phosphate in situ in the water sample.

12. The apparatus of claim 11, wherein the metal/metal oxide electrode comprises at least one selected from a group consisting of a cobalt/cobalt-oxide electrode, a molybdenum/molybdenum oxide electrode, and a tungsten/tungsten oxide electrode; and/or the counter electrode comprises a stainless-steel electrode.

13. The apparatus of any one of claims 11 or 12, wherein the potentiometric sensor further comprises a reference electrode; optionally wherein, the reference electrode comprises a double junction silver-silver chloride electrode.

14. A kit for field monitoring of water, the kit comprising:

- the apparatus of any one of claims 11 to 13; and
- sandpaper for polishing the potentiometric sensor.

15. The kit of claim 14, wherein the sandpaper comprises silicon carbide.

**FIG. 1A**

**FIG. 1B**

200

Electrically pre-treat a potentiometric sensor — 210

Use the electrically pre-treated potentiometric sensor in a water sample to obtain a phosphate measurement — 220

Clean the sensor — 230

**FIG. 2**

300

**FIG. 3A**

302

**FIG. 3B**

310

**FIG. 3C**

320

**FIG. 3D**

330

**FIG. 3E**

340

**FIG. 3F**

FIG. 4A

FIG. 4B

EP 4 137 809 A1

**FIG. 5A**

**FIG. 5B**

**FIG. 6**

FIG. 7

FIG. 8A

FIG. 8B

FIG. 8C

FIG. 8D

FIG. 9

Europäisches
Patentamt

European
Patent Office

Office européen
des brevets

# EUROPEAN SEARCH REPORT

Application Number

EP 22 19 0876

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/158682 A1 (CHANG WOO-JIN [US] ET AL) 21 May 2020 (2020-05-21) | 1-3,8,9, 11-13 | INV. G01N27/333 |
| Y | * the whole document * | 4-7,10, 14,15 | G01N33/18 G01N27/416 G01N27/42 |
| Y | XU KEBIN ET AL: "Phosphate ion sensor using a cobalt phosphate coated cobalt electrode", ELECTROCHIMICA ACTA, vol. 282, 1 August 2018 (2018-08-01), pages 242-246, XP055981303, AMSTERDAM, NL ISSN: 0013-4686, DOI: 10.1016/j.electacta.2018.06.021 * abstract * * paragraph "2.2. Apparatus" * * paragraph "2.3. Preparation of Co modified electrodes" * * paragraph "2.4. Electrochemical measurement" * * figures * | 4-7,10, 14,15 | |
| A | ZHU LEI ET AL: "A potentiometric cobalt-based phosphate sensor based on screen-printing technology", FRONTIERS OF ENVIRONMENTAL SCIENCE, vol. 8, no. 6, 1 December 2014 (2014-12-01), pages 945-951, XP055981311, Beijing ISSN: 2095-2201, DOI: 10.1007/s11783-013-0615-z Retrieved from the Internet: URL:https://link.springer.com/content/pdf/ 10.1007/s11783-013-0615-z.pdf> * abstract * * paragraph "2.3 Pretreatment and measurement" * | 1-15 | TECHNICAL FIELDS SEARCHED (IPC) G01N |

-/--

The present search report has been drawn up for all claims

| Place of search | Date of completion of the search | Examiner |
|---|---|---|
| Munich | 15 November 2022 | Ruchaud, Nicolas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................
& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

**EP 22 19 0876**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CHEN GUOZHU ET AL: "Assessment of a Solid-State Phosphate Selective Electrode Based on Tungsten", JOURNAL OF THE ELECTROCHEMICAL SOCIETY, vol. 165, no. 16, 1 January 2018 (2018-01-01), pages B787-B794, XP055981276, ISSN: 0013-4651, DOI: 10.1149/2.0101816jes Retrieved from the Internet: URL:https://iopscience.iop.org/article/10.1149/2.0101816jes/pdf> * abstract * * page B787, column 1, paragraph 3 * ----- | 1-15 | |

TECHNICAL FIELDS SEARCHED (IPC)

The present search report has been drawn up for all claims

| Place of search | Date of completion of the search | Examiner |
|---|---|---|
| Munich | 15 November 2022 | Ruchaud, Nicolas |

EPO FORM 1503 03.82 (P04C01)

## ANNEX TO THE EUROPEAN SEARCH REPORT
## ON EUROPEAN PATENT APPLICATION NO.

EP 22 19 0876

This annex lists the patent family members relating to the patent documents cited in the above-mentioned European search report.
The members are as contained in the European Patent Office EDP file on
The European Patent Office is in no way liable for these particulars which are merely given for the purpose of information.

15-11-2022

| Patent document cited in search report | Publication date | Patent family member(s) | Publication date |
|---|---|---|---|
| US 2020158682 A1 | 21-05-2020 | US 2020158682 A1<br>WO 2019033034 A1 | 21-05-2020<br>14-02-2019 |

For more details about this annex : see Official Journal of the European Patent Office, No. 12/82

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63260329